(19) Europäisches Patentamt
European Patent Office
Office européen des brevets

(11) Veröffentlichungsnummer: **0 364 765 A2**

# (12) EUROPÄISCHE PATENTANMELDUNG

(21) Anmeldenummer: **89117614.1**

(22) Anmeldetag: **23.09.89**

(51) Int. Cl.5: **C07D 251/30 , C07C 275/40 , C07C 275/62 , C07C 255/42 , C07C 265/12 , C07D 417/10 , C07D 413/10 , C07D 401/10 , C07D 277/64 , C07D 263/56 , A01N 47/38**

(30) Priorität: **08.10.88 DE 3834272**

(43) Veröffentlichungstag der Anmeldung: **25.04.90 Patentblatt 90/17**

(84) Benannte Vertragsstaaten: **AT BE CH DE ES FR GB GR IT LI NL SE**

(71) Anmelder: **BAYER AG**

**D-5090 Leverkusen 1 Bayerwerk(DE)**

(72) Erfinder: **Lindner, Werner, Dr.**
**Märchenstrasse 39**
**D-5000 Köln 80(DE)**
Erfinder: **Haberkorn, Axel, Prof. Dr.**
**Fuhlrottstrasse 99**
**D-5600 Wuppertal 1(DE)**

(54) **Substituierte 1,3,5-Triazintrione, Verfahren zu ihrer Herstellung und ihre Verwendung gegen parasitäre Protozoen.**

(57) Die vorliegende Erfindung betrifft neue substituierte 1,3,5-Triazintrione der allgemeinen Formel (I)

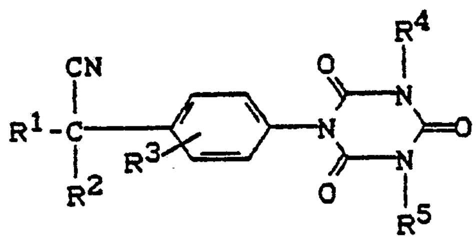

in welcher
$R^1$ für aromatische oder heteroaromatische Reste steht, die gegebenenfalls substituiert sind,
$R^2$ für H, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl, Aralkyl oder Aryl steht, die gegebenenfalls substituiert sind,
$R^5$ für Wasserstoff, gegebenenfalls substituiertes Alkyl, Alkenyl, Alkinyl oder Aralkyl steht.
Sie betrifft ferner Verfahren zu ihrer Herstellung, Zwischenprodukte in diesen Verfahren sowie ihre Verwendung gegen parasitäre Protozoen.

EP 0 364 765 A2

## Substituierte 1,3,5-Triazintrione, Verfahren zu ihrer Herstellung und ihre Verwendung gegen parasitäre Protozoen

Die vorliegende Erfindung betrifft neue substituierte 1,3,5-Triazintrione, Verfahren zu ihrer Herstellung, Zwischenprodukte zur Durchführung dieser Verfahren sowie ihre Verwendung gegen parasitäre Protozoen.

Die Verwendung von substituierten 1,3,5-Triazintrionen zur Bekämpfung von Coccidien ist bekannt. Die Wirkung dieser Verbindungen befriedigt jedoch nicht in jedem Fall.

Die vorliegende Erfindung betrifft

1. Neue substituierte 1,3,5-Triazintrione der allgemeinen Formel (I)

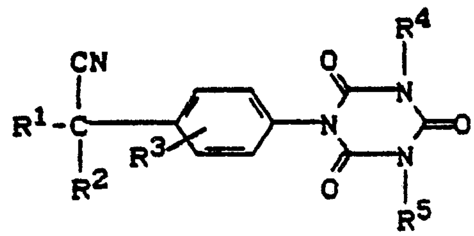

in welcher

$R^1$ für aromatische oder heteroaromatische Reste steht, die gegebenenfalls substituiert sind,

$R^2$ für H, Alkyl, Alkenyl, Alkinyl oder Aralkyl steht, die gegebenenfalls substituiert sind,

$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,

$R^4$ für Wasserstoff, einen geradkettigen, verzweigten oder cyclischen Alkylrest, Alkenyl, Alkinyl, Aralkyl oder Aryl steht, die gegebenenfalls substituiert sind,

$R^5$ für Wasserstoff, gegebenenfalls substituiertes Alkyl, Alkenyl, Alkinyl oder Aralkyl steht.

2. Verfahren zur Herstellung substituierter 1,3,5-Triazintrione der allgemeinen Formel (I)

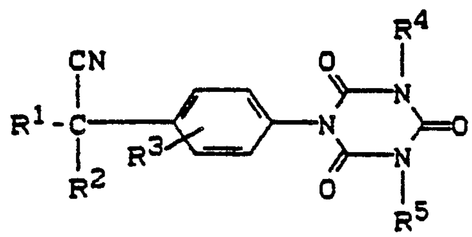

in welcher

$R^1$ für aromatische oder heteroaromatische Reste steht, die gegebenenfalls substituiert sind,

$R^2$ für Wasserstoff, Alkyl, Alkenyl, Alkinyl oder Aralkyl steht, die gegebenenfalls substituiert sind,

$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,

$R^4$ für Wasserstoff, einen geradkettigen, verzweigten oder cyclischen Alkylrest, Alkenyl, Alkinyl, Aralkyl oder Aryl steht, die gegebenenfalls substituiert sind,

$R^5$ für Wasserstoff, gegebenenfalls substituiertes Alkyl, Alkenyl, Alkinyl oder Aralkyl steht,

indem man

a) Verbindungen der Formel (II)

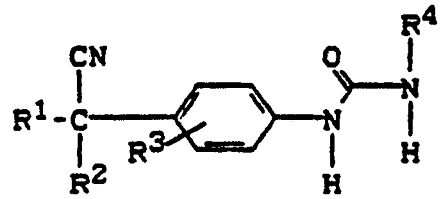

in welcher $R^1$, $R^2$, $R^3$, $R^4$ die oben angegebenen Bedeutungen haben, mit einem substituierten Carbonylisocyanat der Formel (III)

$R^6-\underset{\underset{O}{\|}}{C}-N=C=O$

in der

$R^6$ für ein Halogenatom, eine Alkoxygruppe oder eine Aryloxygruppe steht, umsetzt.

b) Verbindungen der Formel (II) mit Verbindungen der Formel (IV)

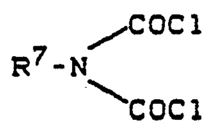

in welcher

$R^7$ für Wasserstoff oder Alkyl steht,

gegebenenfalls in Gegenwart von Säureakzeptoren umsetzt.

c) Verbindungen der Formel (V)

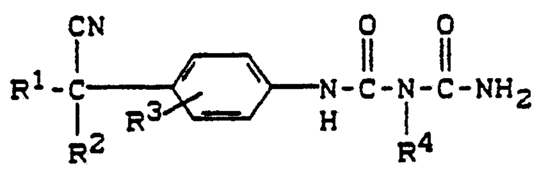

in welcher $R^1$, $R^2$, $R^3$, $R^4$ die oben angegebenen Bedeutungen haben, mit Verbindungen der Formel (VI)

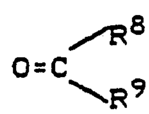

in welcher $R^8$, $R^9$ für eine Alkoxygruppe stehen, gegebenenfalls in Gegenwart von Basen, umsetzt.

d) Verbindungen der Formel (Ia)

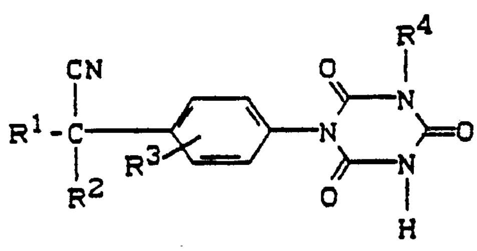

in welcher $R^1$, $R^2$, $R^3$, $R^4$ die oben angegebenen Bedeutungen haben, mit Verbindungen der Formel (VII)

$R^5$ - A

in welcher
$R^5$ für gegebenenfalls substituiertes Alkyl, Alkenyl, Alkinyl, Aralkyl steht und
A für Halogen, $OSO_2$, Alkyl, $OSO_2$-Aryl, $OSO_2$-Halogenalkyl steht.

3. Neue Verbindungen der Formel (II)

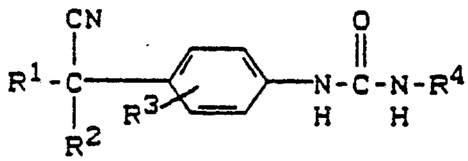

in welcher $R^1$, $R^2$, $R^3$, $R^4$ die oben angegebenen Bedeutungen haben,

4. Verfahren zur Herstellung der Verbindungen der Formel (II) gemäß 3, dadurch gekennzeichnet, daß man

a) Verbindungen der Formel (VIII)

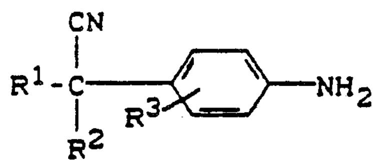

in welcher
$R^1$, $R^2$, $R^3$ die oben genannten Bedeutungen besitzen,
mit Isocyanaten der Formel (IX)
$O=C=N-R^4$
in welcher
$R^4$ die bei den Verbindungen der Formel (I) genannten Bedeutungen besitzt,
umsetzt.

b) Verbindungen der Formel (X)

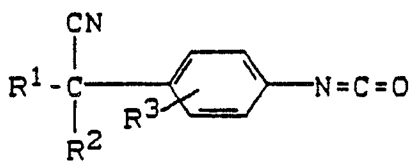

in welcher $R^1$, $R^2$, $R^3$ die bei der Verbindung der Formel (I) genannten Bedeutungen besitzen,
mit Verbindungen der Formel (XI)
$R^4-NH_2$
in welcher
$R^4$ die oben genannten Bedeutungen besitzt.

5. Neue Verbindungen der Formel (V)

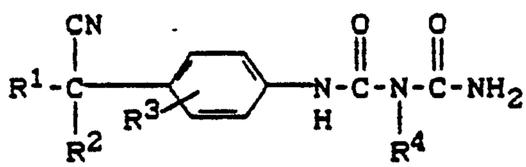

in welcher $R^1$, $R^2$, $R^3$, $R^4$ die oben angegebenen Bedeutungen besitzen.

6. Verfahren zur Herstellung der neuen Verbindungen der Formel (V) gemäß 5, dadurch gekennzeichnet, daß man Verbindungen der Formel (II) mit Phosgen und Ammoniak umsetzt.

7. Neue Verbindungen der Formel (VIII)

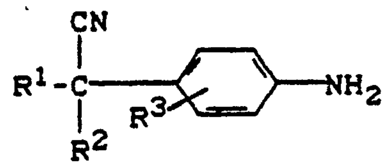

in denen
$R^1$ für Hetero-aromatische Reste außer Thiophen oder für durch Halogenalkylthio oder Halogenalkoxy substituiertes Phenyl steht und
$R^2$, $R^3$ die unter (I) genannten Bedeutungen haben.

8. Verfahren zur Herstellung der neuen Verbindungen der Formel (VIII) gemäß 7., dadurch gekennzeichnet, daß man Verbindungen der Formel (XII)

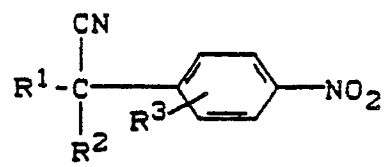

in denen $R^1$, $R^2$, $R^3$ die vorstehend genannten Bedeutungen besitzen,
hydriert.

9. Neue Verbindungen der Formel (X)

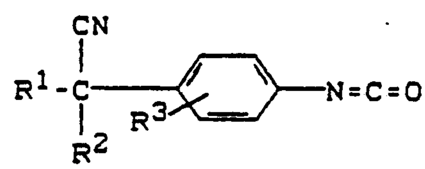

in welcher $R^1$, $R^2$, $R^3$ die unter 4 b) beschriebenen Bedeutungen besitzen.

10. Verfahren zur Herstellung der neuen Verbindungen der Formel (X) gemäß 9., dadurch gekennzeichnet, daß man Verbindungen der Formel (VIII)

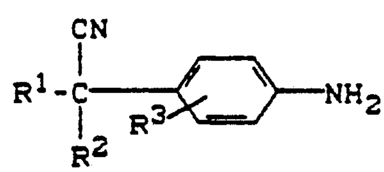

in welcher $R^1$, $R^2$, $R^3$ die unter 4 b) beschriebenen Bedeutungen besitzen
mit Phosgen umsetzt.

11. Neue Verbindungen der Formel (XII)

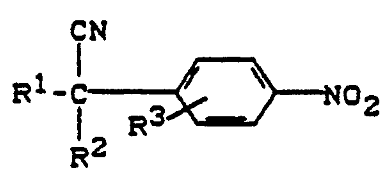

in welcher $R^1$, $R^2$, $R^3$ die unter 7. beschriebenen Bedeutungen besitzen.

12. Verfahren zur Herstellung der neuen Verbindungen der Formel (XII) gemäß 11., dadurch gekennzeichnet, daß man Verbindungen der Formel (XIII)

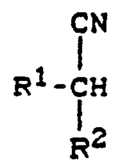

in welcher $R^1$, $R^2$ die unter 7. beschriebenen Bedeutungen besitzen mit Verbindungen der Formel (XIV)

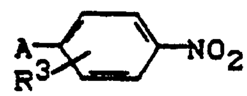

in der $R^3$ die unter 1. beschriebene Bedeutung hat und A für Halogen steht, umsetzt.

Die Verbindungen der Formel (I) sowie ihre Salze mit Säuren oder Basen sind hervorragend zur Bekämpfung parasitischer Protozoen geeignet.

Bevorzugte Verbindungen der Formel (I) sind Verbindungen, in denen
$R^1$ für gegebenenfalls durch Halogen, Alkyl, Cyano, Nitro, O-Alkyl, S-Alkyl, Halogenalkyl, Halogenalkoxy, Halogenalkylthio, Halogenalkylsulfinyl, Halogenalkylsulfonyl, substituiertes Thiazolyl, Oxazolyl, Benzthiazolyl, Benzoxazolyl, Pyridinyl, Pyrimidinyl, Pyrazinyl, Indolyl, Benzimidazolyl, Phenyl oder Naphthyl steht,
$R^2$ für H oder Alkyl steht,
$R^3$ für einen oder mehrere, gleiche oder verschiedene Reste der Gruppe Halogen, Alkyl, Halogenalkyl, Alkoxy, Halogenalkoxy, Alkylthio, Halogenalkylthio oder Cyano steht,
$R^4$ für Wasserstoff, Alkyl, Alkenyl oder Alkinyl steht, die gegebenenfalls durch Halogen, Halogenalkyl, Alkoxy, Alkylthio, Aryloxy, Arylthio oder Aryl substituiert sind,
$R^5$ für Wasserstoff oder Alkyl steht.

Besonders bevorzugt sind Verbindungen der Formel (I), in welcher
$R^1$ für Phenyl, Pyridyl, Benzthiazolyl steht, die durch einen oder mehrere gleiche oder verschiedene Reste der Gruppe Halogen, $C_{1-4}$-Alkyl, $C_{1-4}$-Halogenalkyl, $C_{1-4}$-Alkoxy, $C_{1-4}$-Alkylthio, $C_{1-4}$-Halogenalkoxy, $C_{1-4}$-Halogenalkylthio, Cyano, $C_{1-4}$-Alkoxycarbonyl, $C_{1-4}$-Alkylsulfinyl, $C_{1-4}$-Alkylsulfonyl, $C_{1-4}$-Halogenalkylsulfinyl, $C_{1-4}$-Halogenalkylsulfonyl substituiert ist,
$R^2$ für H oder $C_{1-4}$-Alkyl steht,
$R^3$ für einen oder mehrere gleiche oder verschiedene Reste der Gruppe Wasserstoff, Halogen, $C_{1-4}$-Alkyl, $C_{1-4}$-Halogenalkyl steht,
$R^4$ für Wasserstoff oder $C_{1-4}$-Alkyl steht,
$R^5$ für Wasserstoff oder $C_{1-4}$-Alkyl steht.

Ganz besonders bevorzugt sind Verbindungen der Formel (I), in welcher
$R^1$ für Phenyl steht, das gegebenenfalls durch Halogen, insbesondere Chlor, Brom, Fluor, $C_{1-4}$-Halogenalkyl, insbesondere Trifluormethyl, $C_{1-4}$-Halogenalkylthio, insbesondere Trifluormethylthio, $C_{1-4}$-Halogenalkylsulfinyl, insbesondere Trifluormethylsulfonyl, $C_{1-4}$-Halogenalkylsulfonyl, insbesondere Trifluormethylsulfinyl, $C_{1-4}$-Halogenalkoxy, insbesondere Trifluormethoxy, $C_{1-4}$-Alkyl, insbesondere Methyl, substituiert sind,
$R^2$ für H oder $C_{1-4}$-Alkyl, insbesondere Methyl steht,
$R^3$ für Halogen, insbesondere Brom, Chlor, Fluor, $C_{1-4}$-Alkyl, insbesondere Methyl, Halogenalkyl, insbesondere Trifluormethyl steht,
$R^4$ für $C_{1-4}$-Alkyl, insbesondere Methyl, steht,
$R^5$ für Wasserstoff steht.

Als Einzelverbindungen seien genannt:

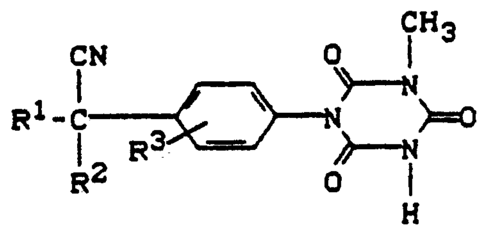

| $R^1$ | $R^2$ | $R^3$ |
|---|---|---|
| 4-Cl-Phenyl | H | 3,5 $Cl_2$ |
| 4-$SCF_3$-Phenyl | H | 3,5 $Cl_2$ |
| 4-$OCF_3$-Phenyl | H | 3,5 $Cl_2$ |
| 4-$SOCF_3$-Phenyl | H | 3,5 $Cl_2$ |
| 4-$SO_2$ $CF_3$-Phenyl | H | 3,5 $Cl_2$ |
| 4-$CF_3$-Phenyl | H | 3,5 $Cl_2$ |
| 3-Cl-4$CF_3$-Phenyl | H | 3,5 $Cl_2$ |
| 3,4-Cl-Phenyl | H | 3,5 $Cl_2$ |

Weiterhin seien die folgenden Verbindungen genannt:

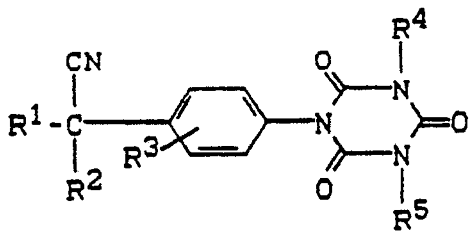

| $R^1$ | $R^2$ | $R^3$ | $R^4$ | $R^5$ |
|---|---|---|---|---|
| 2-Benzthiazolyl | H | 3-Cl | $CH_3$ | H |
| 2-(6-Cl-Benzthiazolyl) | H | 3-Cl | $CH_3$ | H |
| 2-(5,6-Cl-Benzthiazolyl) | H | 3-Cl | $CH_3$ | H |
| 2-Benzoxazolyl | H | 3-Cl | $CH_3$ | H |
| 2-Pyridinyl<br>3-Pyridinyl | H | 3-Cl | $CH_3$ | H |
| 4-Cl-Phenyl | H | $3-CH_3$ | $CH_3$ | H |
| 4-Cl-Phenyl | H | $3,5-CH_3$ | $CH_3$ | H |
| 4-Cl-Phenyl | H | 3-Cl | $CH_3$ | H |
| 4-Cl-Phenyl | H | $3-Cl,5-CH_3$ | $CH_3$ | H |
| 4-Cl-Phenyl | H | $3,5-Br_2$ | $CH_3$ | H |
| 4-Cl-Phenyl | H | $3-CF_3$ | $CH_3$ | H |
| 4-Cl-Phenyl | H | $3,5-Cl_2$ | $C_2H_5$ | H |
| 4-Cl-Phenyl | H | $3-CH_3$ | $-C_2H_5$ | H |
| $4-CF_3$-Phenyl | H | $3-CH_3$ | $CH_3$ | H |
| $4-CF_3$-Phenyl | H | $3,5-CH_3$ | $CH_3$ | H |
| $4-CF_3$-Phenyl | H | 3-Cl | $CH_3$ | H |
| $4-CF_3$-Phenyl | H | $3-Cl,5-CH_3$ | $CH_3$ | H |
| $4-CF_3$-Phenyl | H | 3,5-Br | $CH_3$ | H |
| $4-CF_3$-Phenyl | H | $3-CF_3$ | $CH_3$ | H |
| $4-CF_3$-Phenyl | H | $3,5-Cl_2$ | $C_2H_5$ | H |
| $4-CF_3$-Phenyl | H | $3-CH_3$ | $C_2H_5$ | H |

| $R^1$ | $R^2$ | $R^3$ | $R^4$ | $R^5$ |
|---|---|---|---|---|
| 3,4-$Cl_2$-Phenyl | H | 3-$CH_3$ | $CH_3$ | H |
| 3,4-$Cl_2$-Phenyl | H | 3-Cl | $CH_3$ | H |
| 3,4-$Cl_2$-Phenyl | H | 3-Cl,5-$CH_3$ | $CH_3$ | H |
| 3,4-$Cl_2$-Phenyl | H | 3,5-$Br_2$ | $CH_3$ | H |
| 3,4-$Cl_2$-Phenyl | H | 3-$CF_3$ | $CH_3$ | H |
| 3,4-$Cl_2$-Phenyl | H | 3,5-$Cl_2$ | $C_2H_5$ | H |
| 3,4-$Cl_2$-Phenyl | H | 3-$CH_3$ | $C_2H_5$ | H |
| 2,4-$Cl_2$-Phenyl | H | 3-$CH_3$ | $CH_3$ | H |
| 2,4-$Cl_2$-Phenyl | H | 3-Cl | $CH_3$ | H |
| 2,4-$Cl_2$-Phenyl | H | 3-Cl,5-$CH_3$ | $CH_3$ | H |
| 2,4-$Cl_2$-Phenyl | H | 3,5-$Br_2$ | $CH_3$ | H |
| 2,4-$Cl_2$-Phenyl | H | 3-$CF_3$ | $CH_3$ | H |
| 2,4-$Cl_2$-Phenyl | H | 3,5-$Cl_2$ | $C_2H_5$ | H |
| 2,4-$Cl_2$-Phenyl | H | 3-$CH_3$ | $C_2H_5$ | H |
| 3-Cl-Phenyl | H | 3-$CH_3$ | $CH_3$ | H |
| 3-Cl-Phenyl | H | 3-Cl | $CH_3$ | H |
| 3-Cl-Phenyl | H | 3-Cl,5-$CH_3$ | $CH_3$ | H |
| 3-Cl-Phenyl | H | 3,5-$Br_2$ | $CH_3$ | H |
| 3-Cl-Phenyl | H | 3-$CF_3$ | $CH_3$ | H |
| 3-Cl-Phenyl | H | 3,5-$Cl_2$ | $C_2H_5$ | H |
| 3-Cl-Phenyl | H | 3-$CH_3$ | $C_2H_5$ | H |
| 4-$SCF_3$-Phenyl | H | 3-$CH_3$ | $CH_3$ | H |
| 4-$SCF_3$-Phenyl | H | 3,5-$CH_3$ | $CH_3$ | H |
| 4-$SCF_3$-Phenyl | H | 3-Cl | $CH_3$ | H |

| $R^1$ | $R^2$ | $R^3$ | $R^4$ | $R^5$ |
|---|---|---|---|---|
| 4-$SCF_3$-Phenyl | H | 3-Cl,5-$CH_3$ | $CH_3$ | H |
| 4-$SCF_3$-Phenyl | H | 3,5-$Br_2$ | $CH_3$ | H |
| 4-$SCF_3$-Phenyl | H | 3-$CF_3$ | $CH_3$ | H |
| 4-$SCF_3$-Phenyl | H | 3,5-$Cl_2$ | $C_2H_5$ | H |
| 4-$SCF_3$-Phenyl | H | 3,5-$Cl_2$ | $C_2H_5$ | $CH_3$ |
| 4-$SCF_3$-Phenyl | H | 3,5-$Cl_2$ | $CH_3$ | $CH_3$ |
| 4-$SCF_3$-Phenyl | H | 3-$CH_3$ | $CH_3$ | H |
| 4-$OCF_3$-Phenyl | H | 3,5-$CH_3$ | $CH_3$ | H |
| 4-$OCF_3$-Phenyl | H | 3-$CH_3$ | $CH_3$ | H |
| 4-$SCF_3$-Phenyl | $CH_3$ | 3,5-$Cl_2$ | $CH_3$ | H |
| 4-$OCF_3$-Phenyl | $CH_3$ | 3,5-$Cl_2$ | $CH_3$ | H |
| 4-$OCF_3$-Phenyl | H | 3-Cl | $CH_3$ | H |
| 4-$OCF_3$-Phenyl | H | 3-Cl,5-$CH_3$ | $CH_3$ | H |
| 4-$OCF_3$-Phenyl | H | 3,5-$Br_2$ | $CH_3$ | H |
| 4-$OCF_3$-Phenyl | H | 3-$CF_3$ | $CH_3$ | H |
| 4-$OCF_3$-Phenyl | H | 3,5-$Cl_2$ | $C_2H_5$ | H |
| 4-$OCF_3$-Phenyl | H | 3-Cl | $C_2H_5$ | H |
| 4-CN-Phenyl | H | 3-$CH_3$ | $CH_3$ | H |
| 4-CN-Phenyl | H | 3-Cl | $CH_3$ | H |
| 4-CN-Phenyl | H | 3-Cl,5-$CH_3$ | $CH_3$ | H |
| 4-CN-Phenyl | H | 3,5-$Br_2$ | $CH_3$ | H |
| 4-CN-Phenyl | H | 3-$CF_3$ | $CH_3$ | H |
| 4-CN-Phenyl | H | 3,5-$Cl_2$ | $C_2H_5$ | H |
| 4-CN-Phenyl | H | 3-Cl | $C_2H_5$ | H |

| $R^1$ | $R^2$ | $R^3$ | $R^4$ | $R^5$ |
|---|---|---|---|---|
| 3-$OCH_3$-Phenyl | H | 3-$CH_3$ | $CH_3$ | H |
| 4-$OCH_3$-Phenyl | H | 3-Cl | $CH_3$ | H |
| 4-$OCH_3$-Phenyl | H | 3-Cl,5-$CH_3$ | $CH_3$ | H |
| 4-$OCH_3$-Phenyl | H | 3,5-$Br_2$ | $CH_3$ | H |
| 4-$OCH_3$-Phenyl | H | 3-$CF_3$ | $CH_3$ | H |
| 4-$OCH_3$-Phenyl | H | 3,5-$Cl_2$ | $C_2H_5$ | H |
| 4-$OCH_3$-Phenyl | H | 3-Cl | $C_2H_5$ | H |
| 4-$SO_2CF_3$-Phenyl | H | 3-$CH_3$ | $CH_3$ | H |
| 4-$SO_2CF_3$-Phenyl | H | 3,5-$CH_3$ | $CH_3$ | H |
| 4-$SO_2CF_3$-Phenyl | H | 3-Cl | $CH_3$ | H |
| 4-$SO_2CF_3$-Phenyl | H | 3-Cl,5-$CH_3$ | $CH_3$ | H |
| 4-$SO_2CF_3$-Phenyl | H | 3,5-$Br_2$ | $CH_3$ | H |
| 4-$SO_2CF_3$-Phenyl | H | 3-$CF_3$ | $CH_3$ | H |
| 4-$SO_2CF_3$-Phenyl | H | 3,5-$Cl_2$ | $C_2H_5$ | H |
| 4-$SO_2CF_3$-Phenyl | H | 3-Cl | $C_2H_5$ | H |
| 4-$SOCF_3$-Phenyl | H | 3-$CH_3$ | $CH_3$ | H |
| 4-$SOCF_3$-Phenyl | H | 3-Cl | $CH_3$ | H |
| 4-$SOCF_3$-Phenyl | H | 3-Cl,5-$CH_3$ | $CH_3$ | H |
| 4-$SOCF_3$-Phenyl | H | 3,5-$Br_2$ | $CH_3$ | H |
| 4-$SOCF_3$-Phenyl | H | 3-$CF_3$ | $CH_3$ | H |
| 4-$SOCF_3$-Phenyl | H | 3,5-$Cl_2$ | $C_2H_5$ | H |
| 4-$SOCF_3$-Phenyl | H | 3-Cl | $C_2H_5$ | H |
| 2-Benzimidazololy | H | 3-Cl | $CH_3$ | H |
| 2-Indolyl | H | 3-Cl | $CH_3$ | H |

Setzt man bei dem Verfahren 2 a als Verbindung II 2,6-Dichlor-α-(4-trifluormethylthiophenyl)-4-methylureido-phenylacetonitril ein und als Verbindung der Formel (III) Chlorcarbonylisocyanat ein, läßt sich das Verfahren durch folgendes Formelschema beschreiben:

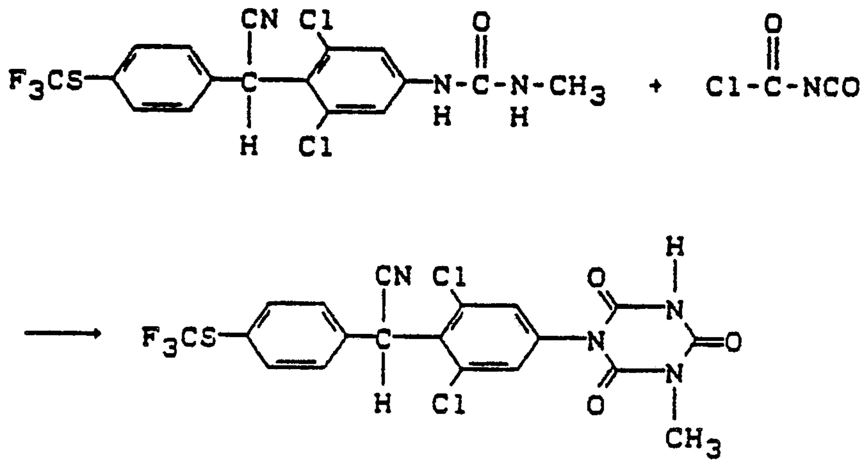

Verbindungen der Formel (II) sind neu. Bevorzugt seien die Verbindungen der Formel (II) genannt, in denen $R^2$ und $R^3$ die bei den Verbindungen der Formel (I) genannten bevorzugten Bedeutungen haben.

Im einzelnen seien folgende Verbindungen der Formel (II) genannt.

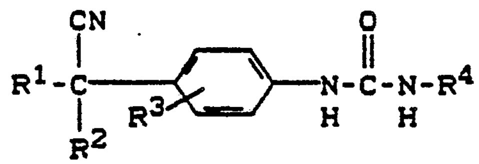

| $R^1$ | $R^2$ | $R^3$ | $R^4$ |
|---|---|---|---|
| 4-Cl-Phenyl | H | 3,5-$Cl_2$ | $CH_3$ |
| 4-$OCF_3$-Phenyl | H | 3,5-$Cl_2$ | $CH_3$ |
| 4-$SOCF_3$-Phenyl | H | 3,5-$Cl_2$ | $CH_3$ |
| 4-$SO_2CF_3$-Phenyl | H | 3,5-$Cl_2$ | $CH_3$ |
| 4-$CH_3$-Phenyl | H | 3,5-$Cl_2$ | $CH_3$ |
| 3,4-$Cl_2$-Phenyl | H | 3,5-$Cl_2$ | $CH_3$ |
| 4-$F_3CO$-Phenyl | H | 3-Cl | $CH_3$ |
| 4-$CF_3$-Phenyl | H | 3-$CH_3$ | $CH_3$ |
| 4-Cl-Phenyl | H | 3-Cl | $CH_3$ |
| 4-Cl-Phenyl | H | 3-$CH_3$ | $CH_3$ |

Die substituierten Carbonylisocyanate der Formel (III) sind bekannt.

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Dimethylacetamid und N-Methyl-pyrrolidon sowie Dimethylsulfoxid, Tetramethylensulfon und Hexamethylphosphorsäuretriamid.

Die Umsetzung erfolgt bei Temperaturen zwischen 20 und 150° C, vorzugsweise zwischen 50 und 120° C.

Das Verfahren wird durchgeführt, indem äquimolare Mengen der Verbindungen der Formel (II) und (III) in einem der angegebenen Verdünnungsmittel zusammengegeben und erhitzt werden. Nach vollendeter Umsetzung wird abgekühlt und der ausgefallene Feststoff abfiltriert, gewaschen und getrocknet.

Setzt man bei dem Verfahren 2 b zur Herstellung der Verbindungen der Formel (I) als Verbindung der Formel (II) 2,6-Dichlor-α-(4-Trifluormethylthiophenyl)-4-methylureido-phenyl-acetonitril ein und als Verbindung der Formel (IV) Bischlorcarbonylmethylamin ein, läßt sich das Verfahren durch folgendes Schema beschreiben.

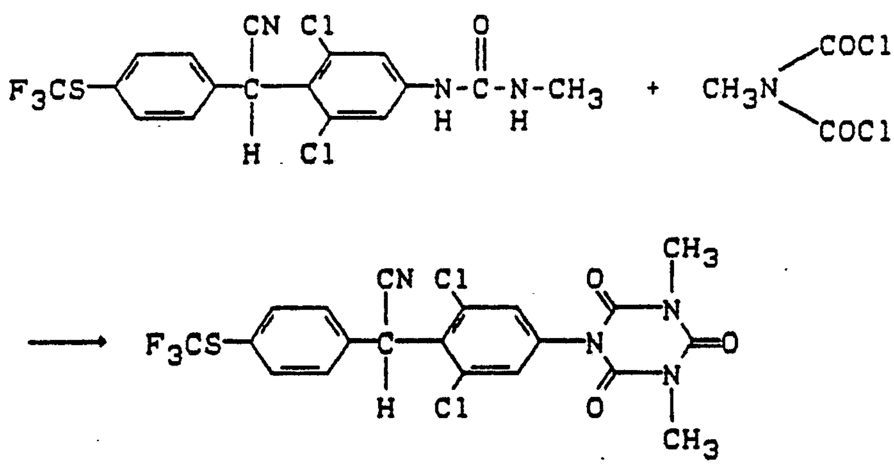

Die Verbindungen der Formel (IV) sind bekannt.

Das Verfahren wird wie unter 2 a beschrieben durchgeführt. Als Verdünnungsmittel finden die beim Verfahren 2 a beschriebenen Verwendung.

Setzt man bei dem Verfahren 2 c als Verbindung der Formel (V) 2,6-Dichlor-α-(4-trifluormethylthiophenyl)-4-dimethyl-diureido-phenylacetonitril und als Verbindung der Formel (VI) Diethylcarbonat ein, läßt sich das Verfahren durch folgendes Schema beschreiben:

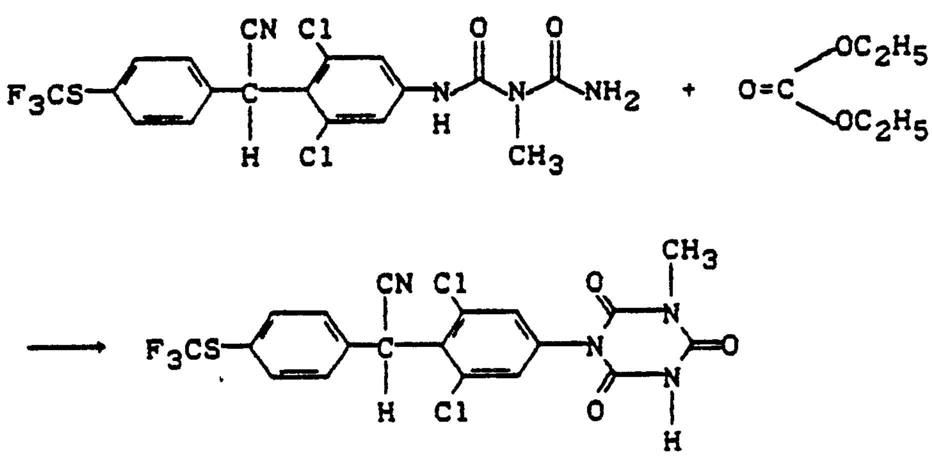

Die Verbindungen der Formel (V) sind neu. Im einzelnen seien folgende Verbindungen der Formel (V) genannt:

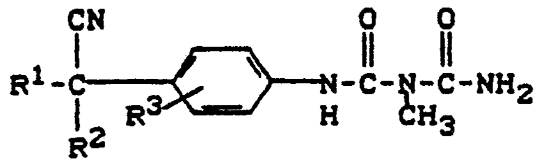

| $R^1$ | $R^2$ | $R^3$ |
|---|---|---|
| 3,4-$Cl_2$-Phenyl | H | 3-Cl |
| 4-$CF_3$-Phenyl | H | 3,5-$Cl_2$ |
| 4-Cl-Phenyl | H | 3,5-$Cl_2$ |
| 4-$OCF_3$-Phenyl | H | 3,5-$Cl_2$ |
| 4-$SCF_3$-Phenyl | H | 3-$CH_3$ |

Das Verfahren wird in Gegenwart von Basem durchgeführt. Als bevorzugte Basen seien genannt die Alkalihydroxide wir Natriumhydroxid, Alkalialkoholate wie Natriumethylat oder organische Basen wir 1,8-Diazabicyclo-[5,4,0]-undec-7-en (DBU).

Das Verfahren wird durchgeführt bei Temperaturen zwischen 10 und 80° C, vorzugsweise zwischen 20 und 50° C, bei Normaldruck oder erhöhtem Druck. Es wird bevorzugt bei Normaldruck gearbeitet. Es kann in Substanz oder in Gegenwart eines Verdünnungsmittels gearbeitet werden. Als Verdünnungsmittel können alle inerten organischen Lösungsmittel eingesetzt werden, die auch bei der Durchführung des Verfahrens 2 a Verwendung finden.

Die Reaktion wird durchgeführt, indem man eine Verbindung der Formel (V) mit einer Verbindung der Formel (VI) in Gegenwart einer Base bei der angegebenen Reaktionstemperatur rührt. Die Menge der Verbindung der Formel Setzt man bei dem Verfahren der Formel 2 d als Verbindung der Formel (Ia) 2,6-Dichlor-α-(4-chlorphenyl)-α-methyl-4-(3-N-methyl-1,3,5-triazin-2,4,6-(1H, 3H, 5H)-trion-phenylacetonitril ein und als Verbindung der Formel (VII) Methyliodid ein, läßt sich das Verfahren durch folgendes Schema beschreiben:

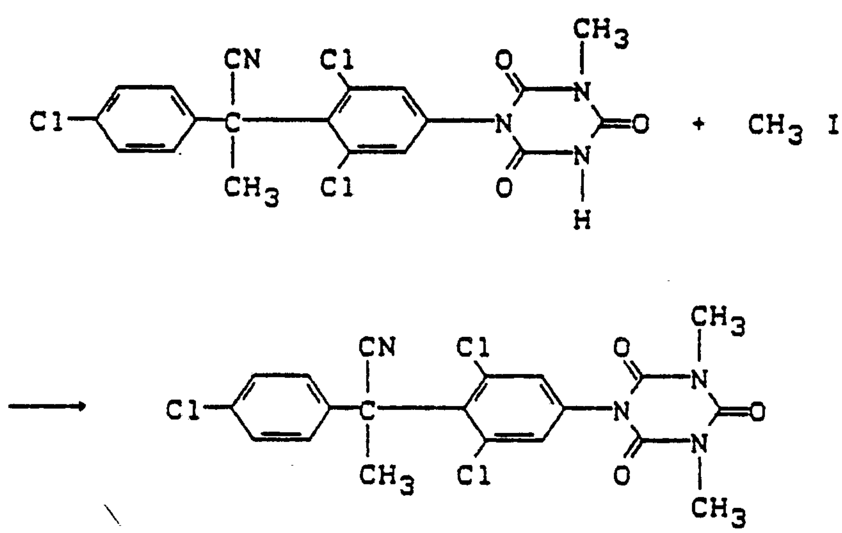

Die Verbindungen der Formel (Ia) sind neu und werden wie bei Verfahren 2 a-c beschrieben dargestellt.

Die Verbindungen der Formel (IV) sind bekannt oder lassen sich nach bekannten Methoden darstellen. Besonders genannt sei Methyliodid, Ethylbromid.

Das Verfahren wird durchgeführt, indem man eine Verbindung der Formel (Ia) in Gegenwart einer Base und eines Verdünnungsmittels mit Verbindungen der Formel (IV) um setzt. Als Verdünnungsmittel können alle inerten organischen Lösungsmittel eingesetzt werden, die auch zur Durchführung von Verfahren 2 a dienen.

Das Verfahren wird in Gegenwart von Basen durchgeführt. Als bevorzugte Basen seien genannt: Alkalihydroxide wie Natriumhydroxid, Alkalialkoholate wie Natriummethylat oder Kaliumbutanolat, Metallhydride wie Natriumhydrid oder organische Basen wie 1,8-Diazabicyclo-[5,40]-undec-7-en (DBU).

Das Verfahren wird durchgeführt bei Normaldruck und Temperaturen zwischen 20 und 140° C.

Die Reaktion wird durchgeführt, indem man äquimolare Mengen der Verbindung der Formel (Ia) und Base zusammengibt, dieses Gemisch mit einer äquimolaren Menge der Verbindung der Formel (IV) versetzt und auf die Reaktionstemperatur erhitzt.

Wird im Verfahren 4 a zur Herstellung der Verbindungen der Formel (II) als Verbindung der Formel (VIII) 4-Amino-2,6-dichloro-α-(4-trifluormethylthiophenyl)-phenylacetonitril und als Verbindung der Formel (IX) Methylisocyanat eingesetzt, läßt sich das Verfahren durch folgendes Formelschema beschreiben:

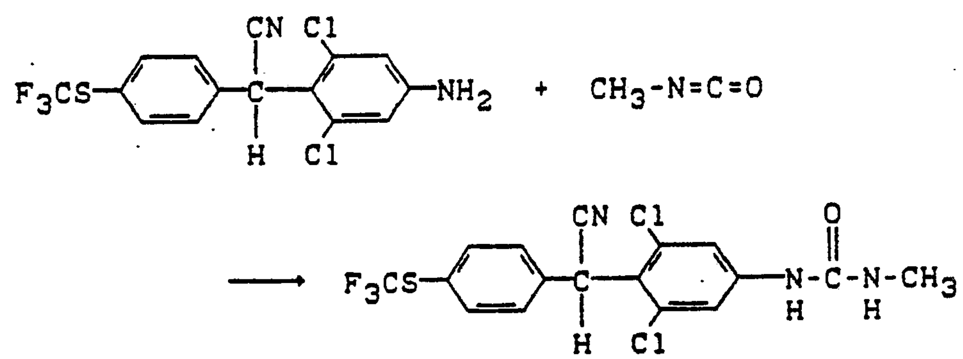

Die Verbindungen der Formel (VIII) sind zum Teil bekannt. Die Verbindungen der Formel (IX) sind bekannt. Als neue Einzelverbindungen der Formel (VIII) seien genannt:

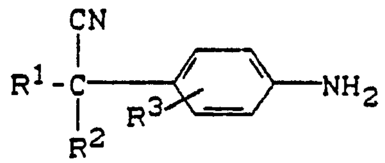

| $R^1$ | $R^2$ | $R^3$ |
|---|---|---|
| 2-(6-Chlorbenzthiazolyl) | H | 3,5-$Cl_2$ |
| 2-Benzthiazolyl | H | 3-Cl |
| 2-Benzthiazolyl | H | 3-$CH_3$ |
| 2-Benzthiazolyl | H | 3,5-$Cl_2$ |
| 2-Benzthiazolyl | H | 3-Cl |
| 2-Indolyl | H | 3-Cl |
| 2-Pyridinyl | H | 3-Cl |
| 3-Pyridinyl | H | 3-Cl |
| 4-$SCF_3$-Phenyl | H | 3-Cl |
| 4-$SCF_3$-Phenyl | H | 3,5-$Cl_2$ |
| 4-$OCF_3$-Phenyl | H | 3,5-$Cl_2$ |
| 4-$OCF_3$-Phenyl | H | 3-Cl |

Das Verfahren wird durchgeführt, indem man eine Verbindung der Formel (VIII) mit einer Verbindung der Formel (IX) in einem inerten Lösungsmittel umsetzt. Als Lösungsmittel können alle inerten organischen Lösungsmittel eingesetzt werden, die auch zur Durchführung von Verfahren 1 a dienen. Zusätzlich genannt sei Pyridin. Das Verfahren wird durchgeführt bei Normal- oder erhöhtem Druck, vorzugsweise bei Normaldruck und bei Temperaturen zwischen 20 und 120° C.

Die Reaktion wird durchgeführt, indem eine Verbindung der Formel (VIII) mit der äquimolaren Menge oder gegebenenfalls einem Überschuß der Verbindung der Formel (IX) in einem Lösungsmittel erhitzt.

Sitzt man bei den Verfahren 4 b zur Herstellung der Verbindungen der Formel (II) als Verbindung der Formel (X) 4-Isocyanato-2,6-dichloro-α-(4-trifluormethylthiophenyl)-phenylacetonitril ein und als Verbindung der Formel (XI) Propylamin ein, läßt sich das Verfahren durch folgendes Formelschema beschreiben:

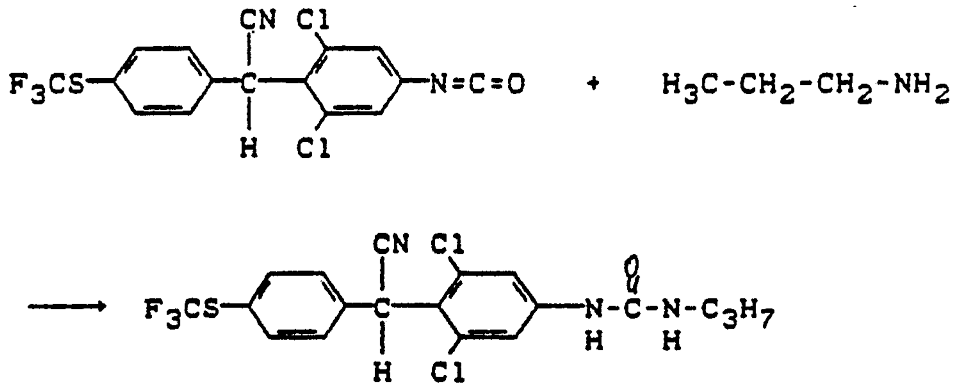

Als Einzelverbindungen der Formel (X) seien genannt:

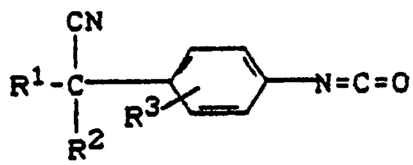

| $R^1$ | $R^2$ | $R^3$ |
|---|---|---|
| Benzthiazolyl | H | 3,5-$Cl_2$ |
| 4-Cl-Phenyl | H | 3-Cl |
| 4-Cl-Phenyl | H | 3,5-$Cl_2$ |
| 4-$OCF_3$-Phenyl | H | 3-$CH_3$ |
| 4-$OCF_3$-Phenyl | H | 3,5-$Cl_2$ |

Das Verfahren wird durchgeführt, indem man eine Verbindung der Formel (X) und eine Verbindung der Formel (XI) gegebenenfalls in Gegenwart eines Verdünnungsmittels erhitzt. Als Verdünnungsmittel finden die bei der Herstellung der Verbindungen (I) aufgeführten Lösungsmittel Verwendung. Zusätzlich genannt sei Pyridin.

Die Umsetzung erfolgt under Normal- oder erhöhtem Druck bei Temperaturen zwischen 50 und 150° C, vorzugsweise zwischen 70 und 110° C.

Die Verbindungen werden in äquimolaren Verhältnissen eingesetzt und das nach abgeschlossener Reaktion als Feststoff anfallende Produkt abfiltriert.

Wird im Verfahren 6 zur Herstellung der Verbindungen der Formel (V) als Verbindung der Formel (II) 2,6-Dichloro-α-(4-chlorophenyl)-4-methylureido-phenylacetonitril eingesetzt, läßt sich das Verfahren durch folgendes Schema beschreiben:

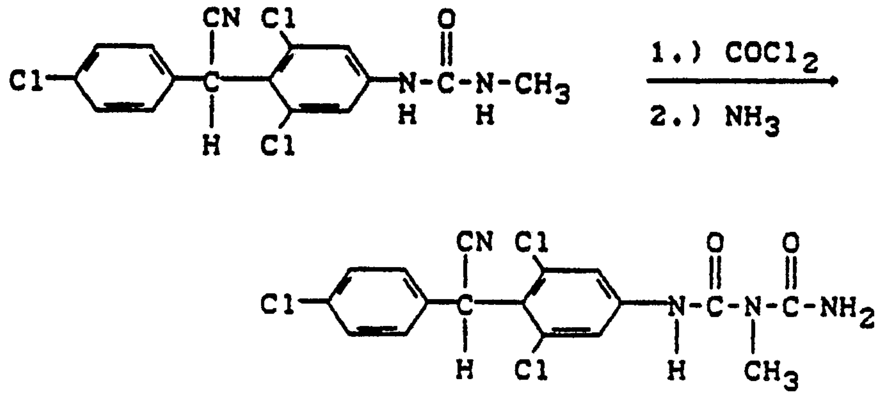


Das Verfahren wird durchgeführt, indem man einen Harnstoff der Formel (II) gegebenenfalls in Gegenwart von Lösungsmitteln zunächst mit Phosgen und dann mit Ammoniak zur Verbindung der Formel (V) umsetzt.

Als Lösungsmittel finden die bei der Herstellung der Verbindungen der Formel (I) angeführten Verwendung.

Phosgen kann gasförmig oder in Lösung in äquimolarer bis 2fach molarer Menge zu der Verbindung der Formel (II) gegeben werden. Ammoniak wird gasförmig so lange durch das Reaktionsgemisch geleitet, bis die Reaktion abgeschlossen ist. Es wird bei Temperaturen zwischen 10 und 80° C, vorzugsweise zwischen 20 und 60° C, gearbeitet. Nach beendeter Reaktion wird abgekühlt und das ausgefallene Produkt abfiltriert.

Die Verfahren 4 a und 6 zur Herstellung der Verbindungen der Formel (V) können vorteilhaft auch im "Eintopfverfahren" durchgeführt werden.

Wird im Verfahren 8 zur Herstellung der neuen Verbindungen der Formel (VIII) als Verbindung der Formel (XII) 4-Nitro-2,6-dichloro-α-(2-benzthiazolyl)-phenylacetonitril eingesetzt, läßt sich das Verfahren durch folgendes Schema beschreiben:

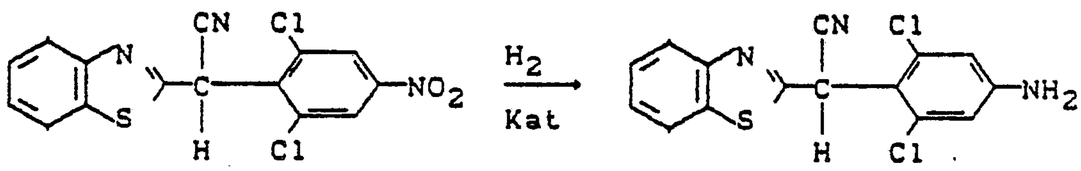


Die Verbindungen der Formel (XII) sind neu unter der Bedingung, daß $R^1$ nicht für Thiophen steht.

Als neue Einzelverbindungen der Formel (XII) seien genannt:

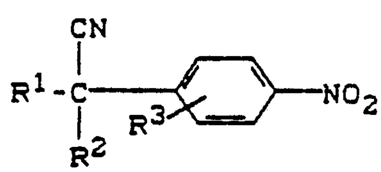

| $R^1$ | $R^2$ | $R^3$ |
|---|---|---|
| 2-(6-Chlorbenzthiazolyl) | H | 3,5-$Cl_2$ |
| 2-Benzthiazolyl | H | 3-Cl |
| 2-Benzthiazolyl | H | 3-$CH_3$ |
| 2-Benzthiazolyl | H | 3,5-$Cl_2$ |
| 2-Benzimidazolyl | H | 3-Cl |
| 2-Indolyl | H | 3-Cl |
| 2-Pyridinyl | H | 3-Cl |
| 3-Pyridinyl | H | 3-Cl |
| 4-$SCF_3$-Phenyl | H | 3,5-$Cl_2$ |
| 4-$OCF_3$-Phenyl | H | 3,5-$Cl_2$ |

Das Verfahren wird durchgeführt, indem man eine Verbindung der Formel (XII) in Gegenwart eines Edelmetallkatalysators hydriert. Die Hydrierung wird gegebenenfalls in Gegenwart eines Verdünnungsmittels bei Normal- oder erhöhtem Druck durchgeführt.

Als Lösungsmittel können vorzugsweise Kohlenwasserstoffe, Alkohole wie Ethanol und Ether wie Tetrahydrofuran verwendet werden. Als Katalysatoren finden Platin, Palladium, Ruthenium, Thodium und bevorzugt Platin Verwendung. Es wird bei Temperaturen zwischen 20 und 130° C, vorzugsweise zwischen 20 und 50° C gearbeitet. Die Katalysatoren werden in 0,01 %igem bis 5 %igem Verhältnis eingesetzt.

Setzt man bei dem Verfahren 10 zur Herstellung der neuen Verbindungen der Formel (X) 2,6-Dichlor-α-(2-benzthiazolyl)-4-amino-phenylacetonitril ein, läßt sich das Verfahren durch folgendes Schema beschreiben:

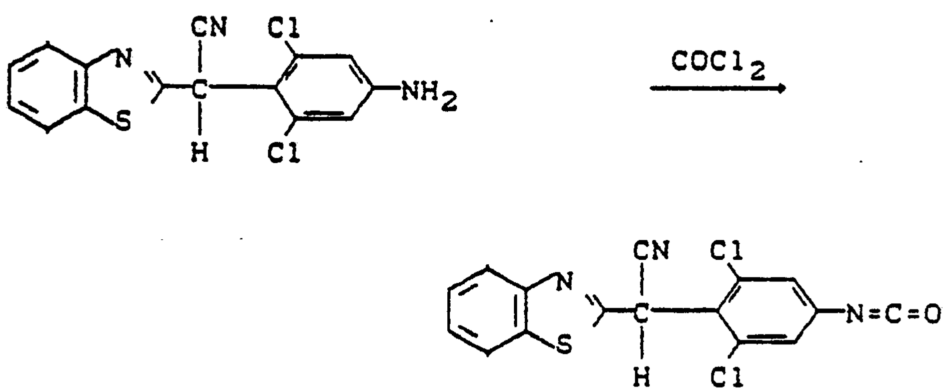


Die Umsetzung der Amine der Formel (VIII) mit Phosgen kann mit oder ohne Verdünnungsmittel erfolgen.

Als Verdünnungsmittel seien genannt insbesondere aliphatische und aromatische, gegebenenfalls halogenierte Kohlenwasserstoffe wie Penten, Hexan, Heptancyclohexan, Petrolether, Benzin, Ligroin, Benzol, Toluol, Methylchlorid, Ethylenchlorid, Chloroform, Tetrachlorkohlenstoff, Chlorbenzol, o-Dichlorbenzol.

Die Umsetzung erfolgt bei -20 - +80° C, bevorzugt bei -10 - +100° C. Es kann bei Normaldruck oder bei erhöhtem Druck gearbeitet werden.

Die Ausgangsstoffe werden in äquimolaren Mengen eingesetzt, bevorzugt ist ein Überschuß an

Phosgen von 2 -3 Mol pro Mol Amin der Formel (VIII).

Die Reaktion wird ohne oder in Gegenwart von Säurebindemitteln durchgeführt. Säurebindemittel sind bevorzugt z. B. tertiäre Amine wir Pyridin, Dimethylamin.

Die Amine der Formel (VIII) werden zu einer Lösung von Phosgen zugegeben und gegebenenfalls unter weiterem Einleiten von Phosgen umgesetzt. Die Umsetzung kann auch ohne Lösungsmittel durchgeführt werden.

Wir im Verfahren 12 zur Herstellung der Verbindungen der Formel (XII) als Verbindung der Formel (XIII) 2-Benzthiazolylacetonitril und als Verbindung der Formel (XIV) 3,4,5-Trichlornitrobenzol eingesetzt, läßt sich das Verfahren durch folgendes Schema beschreiben:

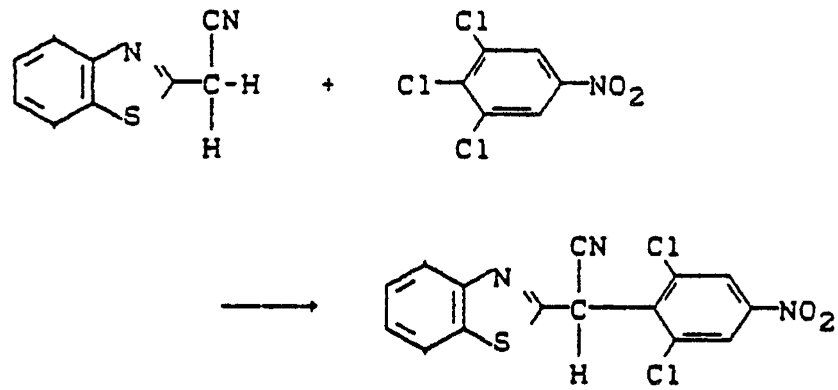

Die Verbindungen der Formel (XII) und (XIII) sind bekannt oder können analog zu bekannten Verfahren hergestellt werden.

Das Verfahren wird durchgeführt, indem man eine Verbindung der Formel (XIII) gegebenenfalls in Gegenwart eines Vewrdünnungsmittels mit Verbindungen der Formel (XIV) umsetzt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldiemthylether, Tetrahydrofuran und Dioxan, Ketone wie Aceton, Methyl- ethyl-, Methyl-isopropyl- und Methylisobutyl-keton, ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Säureakzeptoren durchgeführt.

Als solche seien genannt z.B.:

Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, Metallhydride wie Natriumhydrid, ferner aliphatische, aromatische oder heterocyclische Amine, beispielsweise Triethylamin, Pyridin, 1,5-Diazabiccyclo-[4,3,0]-non-5-en (DBN), 1,8-Diazabicyclo-[5,4,0]-undec-7-en (DBU) und 1,4-Diazabicyclo-[2,2,2]-octan (DABCO).

Als Katalysatoren können Kronenether wie 18 Krone 6 oder quarternäre Ammoniumverbindungen wir Benzyltriethylammoniumchlorid eingesetzt werden.

Die Umsetzung erfolgt bei Temperaturen zwischen 50 und 200° C vorzugsweise zwischen 80 und 160° C bei Normaldruck oder erhöhtem Druck.

Das Verfahren wird durchgeführt indem äquimolare Mengen der Verbindungen der Formel II und III in einem der angegebenen Verdünnungsmittel zusammengegeben und erhitzt werden. Nach vollendeter Umsetzung wird das Reaktionsgemisch mit verdünnter anorganischer Säure (z.B. Salzsäure) angesäuert und der entstehende Niederschlag abfiltriert, gewaschen und getrocknet.

Die Wirkstoffe eignen sich bei günstiger Warmblütertoxizität zur Bekämpfung von parasitischen Protozoen die und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforanas, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Darüber hinaus sind die erfindungsgemäßen Verbindungen wirksam gegenüber verschiedenen zu den Helminthen (Würmern) zählenden Fischparasiten.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major) Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens, (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Workstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös intramuskulär und subcutan verabreicht.

Injektionslösungen werden hergestellt, indem der Wirkstoff in einem geeigneten Lösungsmittel gelöst

wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdikkungsmittel zuzufügen. Verdickungsmittel sind; Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Methyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe auf der Klasse der Benzophenone oder Novantisolsäure.

Hauftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien gennant:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische

Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt, Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.b. Dactylogyrus spp., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Arseln) und Amphipoda (Flohkrebse).

Die Behandlung der Fische erfolgt entweder oral, z. B. über das Futter oder durch Kurzzeitbehandlung, "medizinisches Bad", in das die Fische eingesetzt und in dem sie eine Zeitlang (Minuten bis mehrere Stunden) z. B. beim Umsetzen von einem Zuchtbecken zum anderen gehalten werden.

Es kann aber auch eine vorübergehende oder dauernde Behandlung des Lebensraums der Fische (z. B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Techbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlöslich Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl,

Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basisch Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N′,N′-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

| | | |
|---|---|---|
| a) | Wirkstoff der Formel I | 1 - 10 Gewichtsteile |
| | Sojabohnen-Protein | 49 - 90 Gewichtsteile |
| b) | Wirstoff der Formel I | 0,5 - 10 Gewichtsteile |
| | Benzylalkohol | 0,08 - 1,4 Gewichtsteile |
| | Hydroxypropylmethylcellulose | 0 - 3,5 Gewichtsteile |
| | Wasser | Rest ad 100 |

Zubereitungen zur Anwendung bei "medizinischen Bädern" und zur Teichbehandlung sind z.B. wie folgt zusammengesetzt und hergestellt.

| | | |
|---|---|---|
| c) | 2,5 g Wirkstoff der Formel (I) werden in 100 ml Triethanolamin unter Erwärmen gelöst. | |
| d) | 2,5 g Wirkstoff der Formel (I) | |
| | 12,5 g Milchsäure werden in 100 ml Triethanolamin unter Erwärmen und Rühren gelöst. | |
| e) | 10,0 g Wirkstoff der Formel (I) wird in 100 ml Monoethanolamin gelöst. | |
| f) | Wirkstoff der Formel I | 5,0 g |
| | Propylenglykol | 50,0 g |
| | Natriumcarbonat | 5,0 g |
| | Wasser | ad 100 ml |
| g) | Wirkstoff der Formel I | 5,0 g |
| | Monoethanolamin | 10 g |
| | N-Methylpyrrolidon | ad 100 ml |
| h) | Wirkstoff der Formel I | 2,5 g |
| | Natriumcarbonat | 5,0 g |
| | Polyethylenglykol200 | ad 100 ml |

Der Wirkstoff wird unter Erwärmen im Polyethylenglykol gelöst und Natriumcarbonat darin suspendiert.

Beispiel A

Coccidiose bei Hühnern

9 bis 11 Tage alte Küken wurden mit 40000 sporulierten Oozysten von stark virulenten Stämmen von Eiveria acervulina, E. maxima und E. tenella, den Krankheiteserregern der intestinalen Coccidiose infiziert.

3 Tage vor der Infektion und 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüteten. In der folgenden Tabelle werden die wirksamen Dosen angegeben:

Tabelle 1

| Coccidiose bei Hühnern | | | | | |
|---|---|---|---|---|---|
| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kon-Kontrolle | Blutausscheidung mit dem Kot |
| unbehandelte infizierte Kontrolle | | 2/6 | 100 | 35 | stark |
| 1 | 50 | 0/3 | 0 | 100 | keine |

## Herstellungsbeispiele

### I Beispiele für Verfahren 2a

#### Beispiel 1

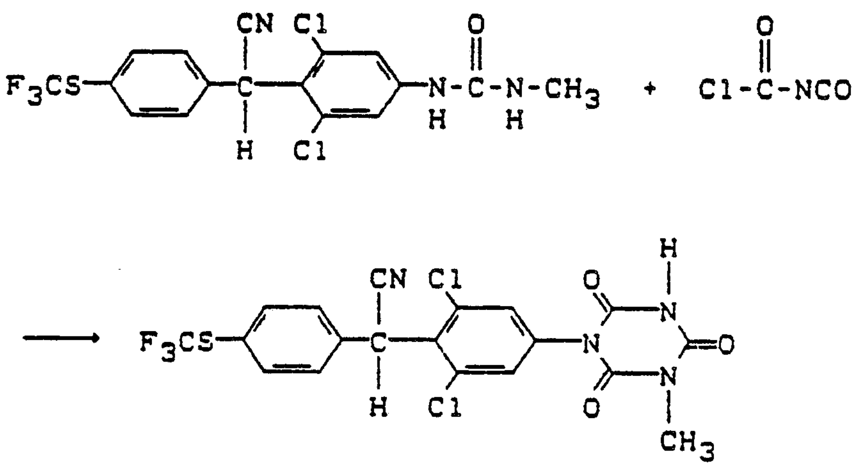

8 g (0,027 mol) 2,6-Dichloro-α-(4-trifluormethylthiophenyl)-4-methylureido-phenylacetonitril werden in 130 ml trockenem Toluol suspendiert und mit 3,6 g (0,035 mol) Chlorcarbonylisocyanat versetzt. Man rührt 3 h bei 60° C, läßt abkühlen, saugt ausgefallenes Produkt ab und wäscht gut mit Cyclohexan nach. Man erhält so 8,5 g (91 % d. Th.) Triazintrion.

Analog werden hergestellt:

#### Beispiel 2

2,6-Dichlor-α-(4-chlorphenyl)-4-(3N-methyl-1,3,5-triazin-2,4,6-(1H,3H,5H)-trion)-phenylacetonitril

#### Beispiel 3

2,6-Dichlor-α-(3,4-Dichlorphenyl)-4-(3N-methyl-1,3,5-triazin-2,4,6-(1H,3H,5H)-trion)-phenylacetonitril

#### Beispiel 4

2,6-Dichlor-α-(4-trifluormethoxyphenyl)-4-(3N-methyl-1,3,5-triazin-2,4,6-(1H,3H,5H)-trion)-phenylacetonitril

### II Beispiel für Verfahren 2b

#### Beispiel 5

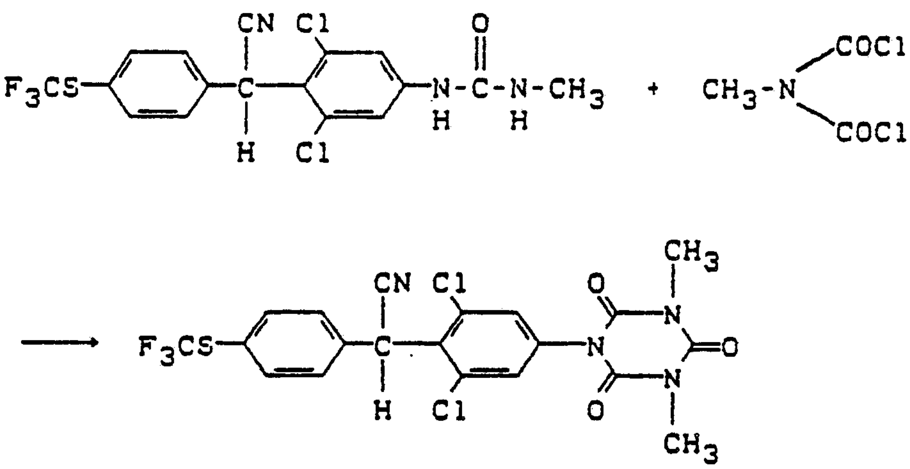

7,4 g (0,017 mol) 2,6-Dichlor-α-(4-trifluormethylthiophenyl)-4-methylureido-phenylacetonitril werden in 100 ml Chlorbenzol suspendiert. Hierzu tropft man 2,8 g (0,018 mol) Bischlorcarbonylamin in 20 ml Chlorbenzol. Man erhitzt auf 110° C und rührt 3 h bei dieser Temperatur nach. Man läßt abkühlen, saugt den Feststoff ab und wäscht mit Cyclohexan nach. Man erhält so 5,9 g (67 % d. Th.) Triazintrion.

III Beispiel für Verfahren 2c

Beispiel 6

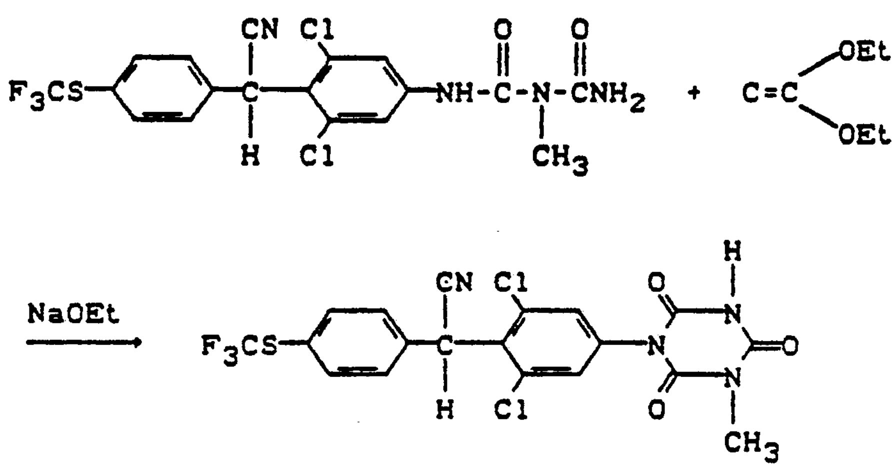


6,3 g (0,014 mol) 2,6-Dichlor-α-(4-trifluormethylthiophenyl)-4-methylbisureido-phenylacetonitril werden in 20 ml Diethylcarbonat suspendiert und 4 h bei 20° C gerührt. Nach Abschluß der Reaktion setzt man Wasser zu und neutralisiert mit verdünnter HCl. Das Reaktionsgemisch wird eingeengt und ausgefallener Feststoff abgesaugt. Man erhält so 5,8 g (85 % d. Th.) Triazintrion.

IV Beispiel für Verfahren 2d

Beispiel 7

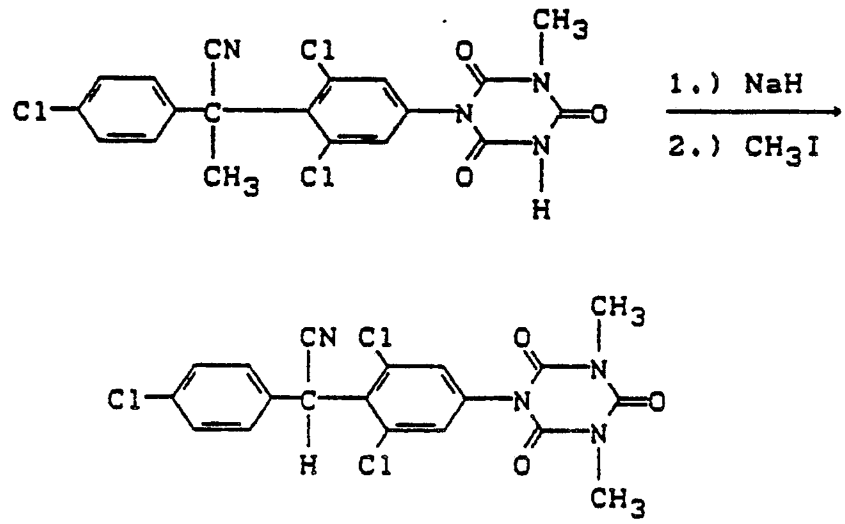


4 g (9,2 mmol) 2,6-Dichlor-α-(4-chlorphenyl)-α-methyl-4-(3-N-methyl-1,3,5-triazin-2,4,6-(1H,3H,5H)-trion-phenylacetonitril werden in 20 ml absolutem DMSO gelöst und mit 0,21 g Natriumhydrid versetzt. Man rührt 30 min bei Raumtemperatur und gibt dann 2 g (14 mmol) Methyliodid in 5 ml DMSO unter Argon zu. Man erwärmt auf 50° C und hält 4 h bei dieser Temperatur. Anschließend wird das Reaktionsgemisch im Vakuum eingeengt und dann mit Wasser versetzt. Man saugt ab und erhält so 3 g (73 %) N,N′-Dimethylverbindung.

V Beispiel für Verfahren 4a

Beispiel Va

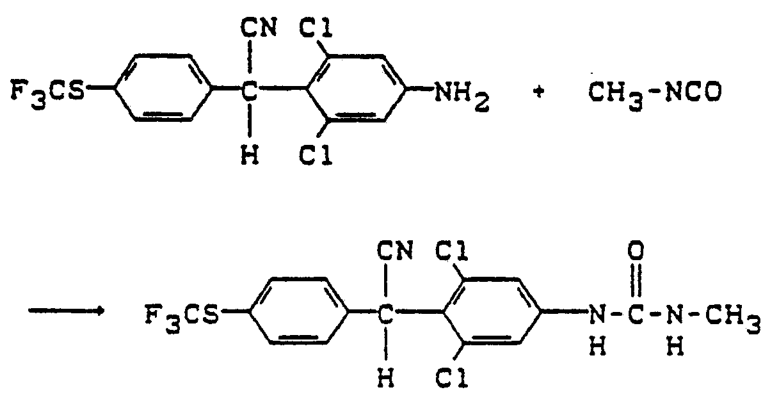

10 g (0,027 mol) 4-Amino-2,6-dichloro-α-(4-trifluormethylthiophenyl)-phenylacetonitril werden in 120 ml trockenem Pyridin gelöst. Hierzu tropft man 2 g (0,035 mol) Methylisocyanat und rührt 15 h bei 30° C nach. Das Reaktionsgemisch wird langsam mit Wasser versetzt und der ausgefallene Feststoff abfiltriert. Man erhält so 10,2 g (88 % d. Th.) Methylharnstoff.

Analog werden hergestellt:

Beispiel Vb

2,6-Dichloro-α-(4-trifluormethoxyphenyl)-4-methylureido-phenylacetonitril

Beispiel Vc

2,6-Dichloro-α-(4-chlorophenyl)-4-methylureido-phenylacetonitril

VI Beispiel zu Verfahren 4b

Beispiel VIa

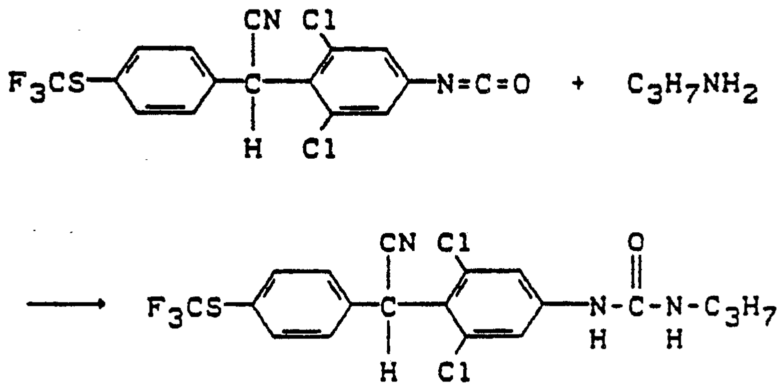

3,5 g (8,1 mmol) 4-Isocyanato-2,6-dichloro-α-(4-trifluormethyl-thiophenyl)-phenylacetonitril werden in 30 ml absolutem Toluol unter Argon gelöst und bei Raumtemperatur mit einer Lösung von 0,6 g (10 mmol) Propylamin in 20 ml Toluol versetzt. Man erwärmt 1 h auf 60° C, dann 2 h auf 90° C. Nach Abziehen der flüchtigen Bestandteile im Vakuum und Umkristallisieren des Rückstandes aus Ethanol erhält man 2,4 g (64 % d. Th.) Propylharnstoff.

VII Beispiel für Verfahren 6

Beispiel VIIa

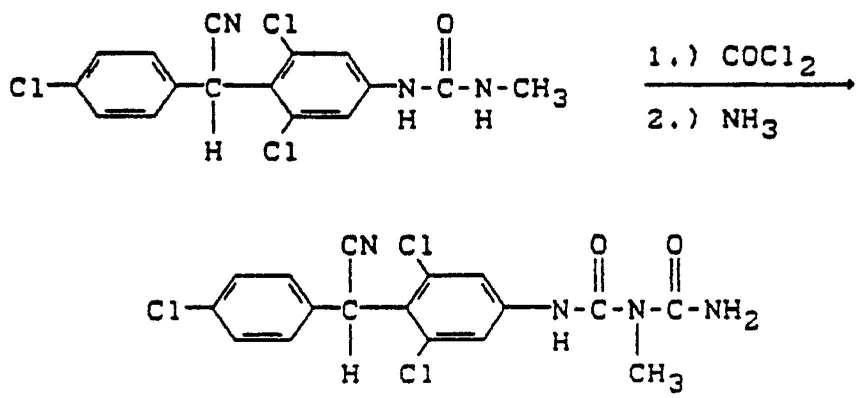


5 g (0,014 mol) 2,6-Dichlor-α-(4-chlorphenyl)-4-methylureido-phenylacetonitril in 70 ml trockenem Toluol werden bei Raumtemperatur mit 12 g 20 %iger (0,025 mol) toluolischer Phosgenlösung versetzt. Man rührt 10 h bei Raumtemperatur nach und erhitzt anschließend 4 h auf 60° C. Nach dem Abkühlen leitet man bei Raumtemperatur bis zur vollständigen Umsetzung des Phosgenaddukts Ammoniak durch das Reaktionsgemisch. Nach Abschluß der Reaktion wird der ausgefallene Feststoff abfiltriert. Man erhält so 4,1 g (72 % d.

Th.) Biuret.

Analog werden hergestellt:

Beispiel VIIb

2,6-Dichlor-α-(trifluormethylthiophenyl)-4-N′-methylbisureido-phenylacetonitril

Beispiel VIIc

2,6-Dichlor-α-(trifluormethoxy)-4-N′-methyl-bisureido-phenylacetonitril

VIII Beispiel für Verfahren 8

Beispiel VIIIa

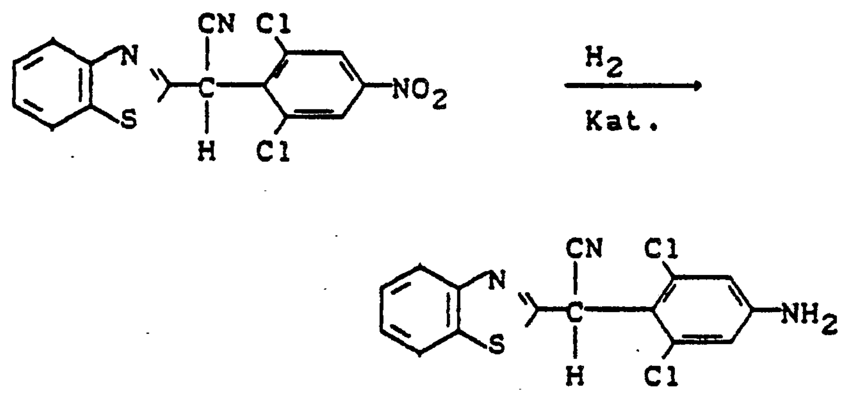


20 g (0,055 mol) 4-Nitro-2,6-dichloro-α-(2-benzthiazolyl)-phenylacetonitril werden in 100 ml Dioxan und 100 ml Ethanol gelöst und bei Normaldruck und Raumtemperatur unter Zugabe von 4 g Pd (10 % auf A-Kohle) hydriert. Nach Abfiltrieren des Katalysators und Abziehen des Lösungsmittels erhält man 16,9 (92 % d. Th.) Amin.

Analog werden hergestellt:

Beispiel VIIIb

4-Amino-2,6-dichloro-α-(2-pyridinyl)-phenyl-acetonitril

Beispiel VIIIc

4-Amino-2,6-dichloro-α-(3-pyridinyl)-phenyl-acetonitril

IX Beispiel für Verfahren 10

Beispiel IXa

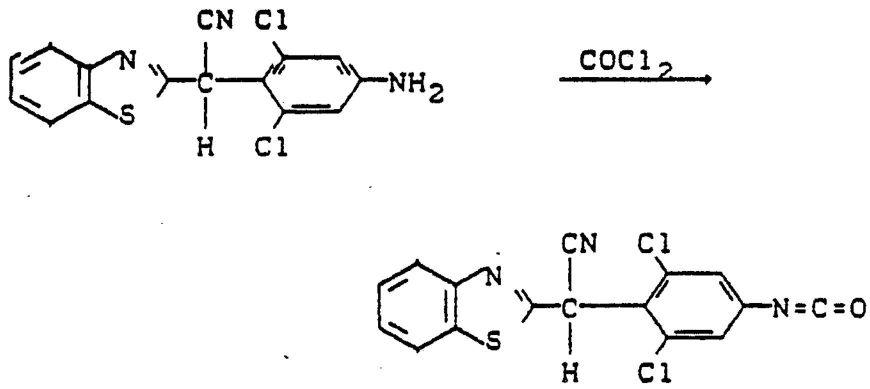


Zu 7,8 g (0,016 mol) Phosgen in Toluol tropft man 4 g (0,012 mol) 2,6-Dichlor-α-(2-benzthiazolyl)-4-aminophenylacetonitril gelöst in 50 ml Toluol. Nach beendetem Zutropfen erwärmt man langsam auf Raumtemperatur, rührt 1 h bei Raumtemperatur und erwärmt dann langsam innerhalb einer weiteren Stunde zum Sieden. Man rührt unter Rückfluß für 2 weitere Stunden, kühlt ab und destilliert die flüchtigen Bestandteile im Vakuum ab und digeriert den Rückstand mit wenig Diethylether. Man erhält so 3,7 g (85 % d. Th.) Isocyanat als Feststoff.

X Beispiel für Verfahren 12

Beispiel Xa

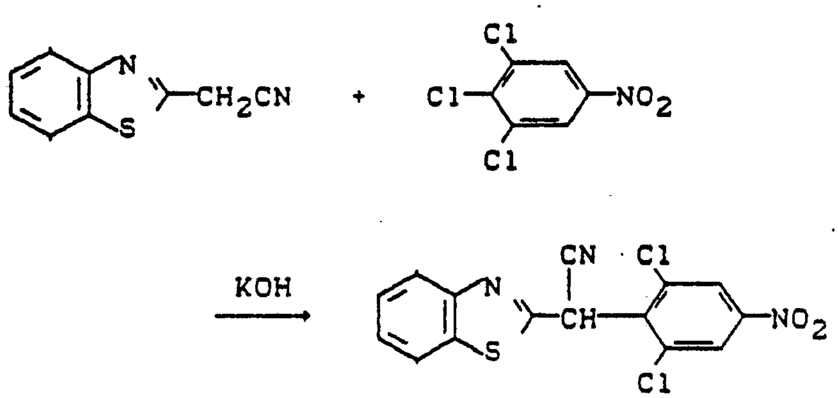


17,4 g (0,1 mol) 2-Benzthiazolylacetonitril 2,6 g (0,1 mol) 3,4,5-Trichlornitrobenzol und 5,6 g (0,1 mol) KOH werden in 500 ml Acetonitril 12 h unter Rückfluß gekocht. Anschließend kühlt man ab, verdünnt mit Wasser und säuert mit HCl an. Der ausgefallene Feststoff wird abfiltriert. Man erhält so 26,9 g (74 % d. Th.) Nitroverbindung.

Analog werden hergestellt:

Beispiel Xb

4-Nitro-2,6-dichloro-α-(2-pyridinyl)-phenylacetonitril

Beispiel Xc

4-Nitro-2,6-dichloro-α-(3-pyridinyl)-phenylacetonitril

**Ansprüche**

1. Substituierte 1,3,5-Triazintrione der allgemeinen Formel (I)

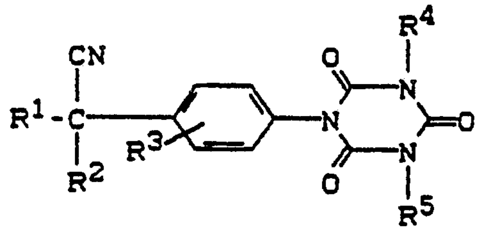

in welcher
$R^1$ für aromatische oder heteroaromatische Reste steht, die gegebenenfalls substituiert sind,
$R^2$ für H, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl, Aralkyl oder Aryl steht, die gegebenenfalls substituiert sind,
$R^5$ für Wasserstoff, gegebenenfalls substituiertes Alkyl, Alkenyl, Alkinyl oder Aralkyl steht.

2. Verfahren zur Herstellung substituierter 1,3,5-Triazintrione der allgemeinen Formel (I)

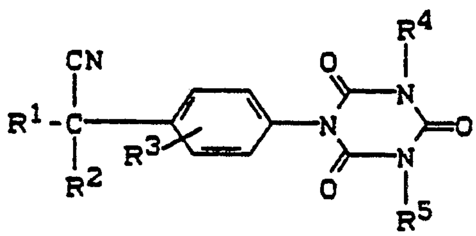

in welcher
$R^1$ für aromatische oder heteroaromatische Reste steht, die gegebenenfalls substituiert sind,
$R^2$ für Wasserstoff, Alkyl, Alkenyl, Alkinyl oder Aralkyl steht, die gegebenenfalls substituiert sind,
$R^3$ für einen oder mehrere, gleiche oder verschiedene Reste der Gruppe Wasserstoff, Halogen, Alkyl, Halogenalkyl, Alkoxy, Alkylthio, Halogenalkoxy, Halogenalkylthio, Cyano, Alkoxycarbonyl, Alkylsulfonyl, Halogenalkylsulfonyl,
$R^4$ für Wasserstoff, einen geradkettigen, verzweigten oder cyclischen Alkylrest, Alkenyl, Alkinyl, Aralkyl oder Aryl steht, die gegebenenfalls substituiert sind,
$R^5$ für Wasserstoff, gegebenenfalls substituiertes Alkyl, Alkenyl, Alkinyl oder Aralkyl steht,
indem man

a) Verbindungen der Formel (II)

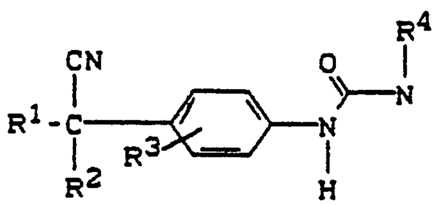

in welcher $R^1$, $R^2$, $R^3$, $R^4$ die oben angegebenen Bedeutungen haben, mit einem substituierten Carbonylisocyanat der Formel (III)

$R^6$- C -N = C = O
‖
O

in der
$R^6$ für ein Halogenatom, eine Alkoxygruppe oder eine Aryloxygruppe steht, umsetzt.

b) Verbindungen der Formel (II) mit Verbindungen der Formel (IV)

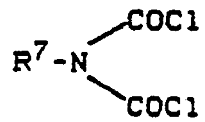

in welcher
$R^7$ für Wasserstoff oder Alkyl steht,
gegebenenfalls in Gegenwart von Säureakzeptoren umsetzt.

c) Verbindungen der Formel (V)

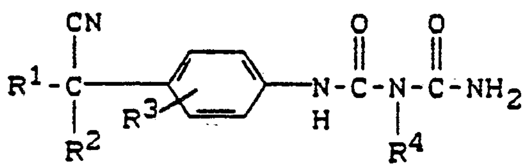

in welcher $R^1$, $R^2$, $R^3$, $R^4$ die oben angegebenen Bedeutungen haben,
mit Verbindungen der Formel (VI)

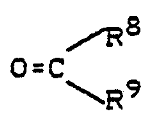

in welcher $R^8$, $R^9$ für eine Alkoxygruppe stehen, gegebenenfalls in Gegenwart von Basen, umsetzt.

d) Verbindungen der Formel (Ia)

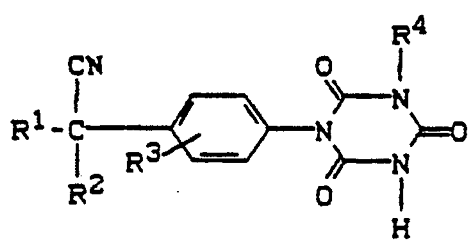

in welcher $R^1$, $R^2$, $R^3$, $R^4$ die oben angegebenen Bedeutungen haben,
mit Verbindungen der Formel (VII)
$R^5$ - A
in welcher
$R^5$ für gegebenenfalls substituiertes Alkyl, Alkenyl, Alkinyl, Aralkyl steht und
A für Halogen, $OSO_2$, Alkyl, $OSO_2$-Aryl, $OSO_2$-Halogenalkyl steht.

3. Verbindungen der Formel (II)

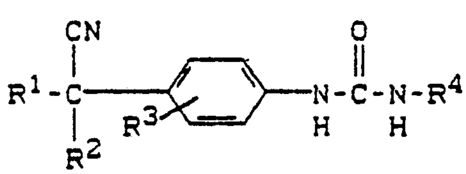

in welcher
$R^1$, $R^2$, $R^3$, $R^4$ die in Anspruch 1 genannten Bedeutungen haben.

4. Verfahren zur Herstellung der Verbindungen der Formel (II) gemäß Anspruch 3, dadurch gekennzeichnet, daß man

a) Verbindungen der Formel (VIII)

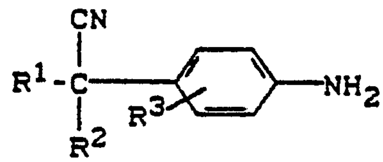

in welcher
$R^1$, $R^2$, $R^3$ die oben genannten Bedeutungen besitzen,
mit Isocyanaten der Formel (IX)
$O=C=N-R^4$
in welcher
$R^4$ die bei den Verbindungen der Formel (I) genannten Bedeutungen besitzt,
umsetzt.

b) Verbindungen der Formel (X)

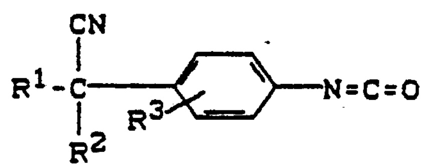

in welcher
$R^1$, $R^2$, $R^3$ die bei den Verbindungen der Formel (I) genannten Bedeutungen besitzen,
mit Verbindungen der Formel (XI)
$R^4-NH_2$
in welcher
$R^4$ die oben genannten Bedeutungen besitzt,
umsetzt.

5. Verbindungen der Formel (V)

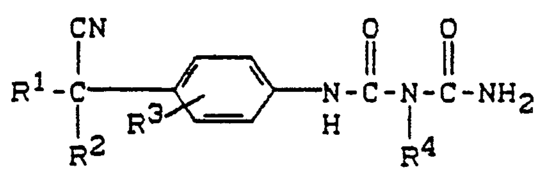

in welcher
$R^1$, $R^2$, $R^3$, $R^4$ die in Anspruch 1 angegebenen Bedeutungen besitzen.

6. Verfahren zur Herstellung der Verbindungen der Formel (V) gemäß Anspruch 5, dadurch gekennzeichnet, daß man Verbindungen der Formel (II) gemäß Anspruch 2 mit Phosgen und Ammoniak umsetzt.

7. Verbindungen der Formel (VIII)

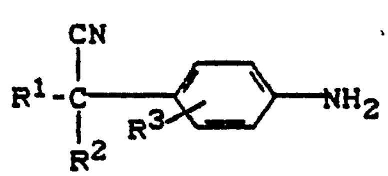

in denen
$R^1$ für Hetero-aromatische Reste außer Thiophen oder für durch Halogenalkylthio oder Halogenalkoxy substituiertes Phenyl steht,
$R^2$, $R^3$ die in Anspruch 2 genannten Bedeutungen haben.

8. Verfahren zur Herstellung der Verbindungen der Formel (VIII) gemäß Anspruch 7, dadurch gekennzeichnet, daß man Verbindungen der Formel (XII)

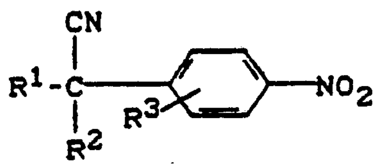

in welcher
$R^1$, $R^2$, $R^3$ die in Anspruch 7 genannten Bedeutungen besitzen,
hydriert.

9. Verbindungen der Formel (X)

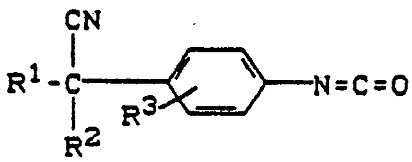

in welcher
$R^1$, $R^2$, $R^3$ die in Anspruch 4 b) genannten Bedeutungen besitzen.

10. Verfahren zur Herstellung der neuen Verbindungen der Formel (X) gemäß Anspruch 9, dadurch gekennzeichnet, daß man Verbindungen der Formel (VIII)

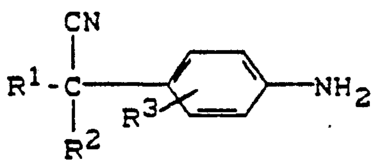

in welcher
$R^1$, $R^2$, $R^3$ die in Anspruch 4 b) genannten Bedeutungen besitzen
mit Phosgen umsetzt.

11. Verbindungen der Formel (XII)

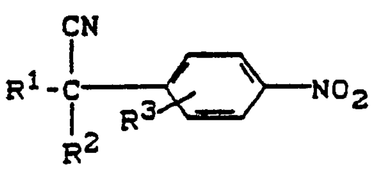

in welcher
$R^1$, $R^2$, $R^3$ die in Anspruch 7 beschriebenen Bedeutungen besitzen.

12. Verfahren zur Herstellung der neuen Verbindungen der Formel (XII) gemäß Anspruch 11, dadurch gekennzeichnet, daß man Verbindungen der Formel (XIII)

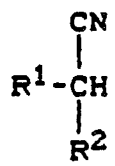

in welcher
$R^1$, $R^2$ die in Anspruch 7 genannten Bedeutungen besitzen
mit Verbindungen der Formel (XIV)

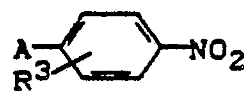

in welcher
$R^3$ die in Anspruch 1 genannten Bedeutungen hat und
A für Halogen steht,
umsetzt.

13. Mittel gegen parasitische Protozoen, gekennzeichnet durch einen Gehalt an mindestens einem 1,3,5-Triazintrion der Formel (I) gemäß Anspruch 1.

14. Verwendung von 1,3,5-Triazintrionen der Formel (I) gemäß Anspruch 1 zur Bekämpfung von parasitischen Protozoen.

15. Verwendung von 1,3,5-Triazintrione der Formel (I) gemäß Anspruch 1 zur Herstellung von Mitteln gegen parasitische Protozoen.